Europäisches Patentamt

European Patent Office

Office européen des brevets

(11) Publication number: 0 466 105 A2

# EUROPEAN PATENT APPLICATION

(21) Application number: 91111418.9

(22) Date of filing: 09.07.91

(51) Int. Cl.5: **A61L 27/00, A61F 2/06**

(30) Priority: 12.07.90 US 551880

(43) Date of publication of application:
15.01.92 Bulletin 92/03

(84) Designated Contracting States:
BE DE ES FR GB IT LU NL SE

(71) Applicant: CORVITA CORPORATION
P.O. Box 025700
Miami, Florida 33102-5700(US)

(72) Inventor: Weldon, Norman R.
8980 S.W. 67th Avenue
Miami, Florida 33156(US)

(74) Representative: KUHNEN, WACKER &
PARTNER
Alois-Steinecker-Strasse 22 Postfach 1553
W-8050 Freising(DE)

(54) Composite biosynthetic graft.

(57) A composite vascular graft including an outer synthetic component positioned over an inner biological component. The biological component is formed by generally shaping extracellular matrix, derived by the processing of naturally-occurring tissue, into a general cylindrical shape having a lumen therethrough. The outer synthetic component is formed by layering non-interwoven strands of synthetic material. The general compliance of the graft may be varied including by varying the layering of the non-interwoven strands and the material from which the strands are made. As implanted, bodily fluids may pass smoothly through the lumen.

FIG-1

## BACKGROUND AND DESCRIPTION OF THE INVENTION

The present invention generally relates to grafts and methods for making same. More particularly, the invention relates to a graft, such as a vascular graft, having an inner component made from biological material and an outer component made from synthetic material. The inner component is formed by the shaping of processed naturally-occurring tissue into an elongated cylindrical shape having a lumen therethrough. Non-interwoven strands of biocompatible synthetic material natural are layered to form an outer component. With the outer component properly positioned over the inner component, advantageously the outer synthetic component adds strength to the graft without compromising elasticity while the inner biological component improves the biocompatibility with bodily fluid flowing through the lumen thereof.

Grafts made entirely of synthetic materials are widely used as replacements for diseased arteries or veins. Their wide use, however, belies the variety of problems from which some of the conventional grafts suffer. Grafts made from wholly synthetic and not generally biocompatible materials prompt immunological and thrombogenic reactions from the host in which the graft is implanted. Whether, and the degree to which the host's system reacts to the graft depends on the particular material from which the graft is made and the structure of the graft. Non-hemocompatibility causes clotting and resultant occlusion in and around the graft. Clotting is a particularly serious problem because of the possible resultant formation of emboli. Occlusion is a progressively more frequent problem and occurs when the lumen of the vascular graft is reduced sufficiently and/or the graft is used in an area of the body, such as replacement for a portion of the venous system, that typically has low blood flow.

Conventional synthetic grafts, again because of the materials from which the grafts are made or because of the graft's structure, also may not facilitate the ingrowth of cells and tissue within the graft wall. Accordingly, incorporation of the graft into the host's tissue is not fully achieved and the graft may occlude.

Another problem with some conventional wholly-synthetic grafts is that their compliance is generally different from that of the walls of the tissue to which they are connected. Blood vessels are not straight, rigid tubes but conduits having walls made of varying, generally elastic materials whose compliance resultingly varies. Compliance of a blood vessel is the ratio of the change in the vessel's diameter to the change in pressure within the vessel. The diameter of a compliant blood vessel will increase and decrease with every heart beat. The use of grafts having walls less compliant than that of the surrounding tissue walls is problematic in that conditions, such as intimal hyperplasia and stenotic narrowing, may develop. The use of grafts having walls too compliant is also problematic in that a portion of the graft wall may balloon - that is, develop an aneurysm - and/or rupture after implantation due to the weakening of the graft in response to the varying pressure.

A demand therefore is present for a graft which is biocompatible and has a compliance which can be adjusted to match that of the host vessel to which it is joined during implantation. The present invention satisfies this demand.

The present invention includes a two-component system, one component of which, an inner component, is made of a biological material which may be processed in the manner detailed in U.S. Patent Nos. 4,801,299 to Brendel et al. and/or 4,776,853 to Klement, et al. These patents are incorporated by reference thereto. By the Klement et al. patented technique, and by the Brendel et al. patented techniques, natural tissue may be processed so that the extracellular matrix of the tissue is retained intact. The other component of the present two-component system, an outer component, is made of synthetic materials of the type detailed in U.S. Patent No. 4,355,426 to MacGregor and, in part, in U.S. Patent No. 4,743,252 to Martin and MacGregor. The outer component may be fabricated in the manner detailed in U.S. Patent No. 4,475,972 to Wong. As a complement to this fabrication method, or wholly separate from it, the outer component may be made according to method detailed in U.S. Patent No. 4,738,740 to Pinchuk and Martin. These patents are incorporated by reference hereinto. Utilizing these materials and this technique, non-interwoven strands of synthetic material, such as polyurethane, may be layered directly onto the inner biological component. Alternatively, the outer component may be formed by the same layering technique but separate from the inner biological component. Subsequently, the outer component is positioned over the inner component and joined, for example, by mechanical means, such as with sutures, or by adhesive means.

The two component system of the present invention provides a number of advantages over conventional grafts. Because the inner surface of the two-component system is lined with biological material processed from natural tissue, the likelihood that the graft will prompt an immunological and/or thrombogenic reaction is lessened. Accordingly, the need to administer antithrombotic or other such drugs to prevent, for example, the formation of microthrombi or microocclusions in and around the graft is lessened.

Furthermore, as the natural lining of the lumen formed within the inner biological component provides a smooth and non-thrombogenic surface over which the blood may flow, the naturally laminar flow of blood is not impeded. A graft having a lumen made from rough materials, or materials which induce an immunological or thrombogenic reaction, and thereby the formation of microthrombi or microoclusions, produces a turbulent flow, thereby impeding the flow of and the transport of blood to peripheral areas.

The use of compliant synthetic materials to make the outer component and the use of processed tissue including collagen, elastin, and basement membrane to make the inner component advantageously, not only allows a compliant hemocompatible graft to be made, but also allows one to be made whose compliance may be adjusted to match the tissue to which the graft is implanted.

Adjustable compliance is an important added advantage which the present invention has in comparison to conventional grafts. This significance of this advantage is recognizable only when the degree to which blood vessels naturally vary in compliance is fully understood. The variation in the compliance of natural tissue is largely dictated by functional considerations. To illustrate, one purpose of the venous system is to function as the body's blood reservoir. To achieve this storage capacity, the vessels of the venous system must be very compliant and be able to distend sufficiently in response to a possible greater volume of blood sent into the system because of changes in the arterial blood pressure. On the other hand, one purpose of the arterial system is to function as the body's pressure reservoir. The arterial system can maintain the pressure needed, for example, to force blood into the small-diameter arterioles and throughout the microcirculatory bed only if the arteries are comparatively less compliant and do not dramatically change in size in response to small changes in pressure as the vessels of the venous system do.

The variation in compliance is found not only between the two main blood vessel systems but also within the systems. For example, in general, the arterial vessels closer to the heart are more elastic and contain a thicker tunica media (i.e., more circular and longitudinal smooth muscle) and a thicker tunica intima (i.e., a thicker endothelial layer and more elastic fibers) than those farther from the heart. This intrasystem variation is necessary because the arteries close to the heart must be sufficiently elastic and be able to distend in response to the great volume of blood ejected into them by the heart yet be able to recoil elastically so as to maintain the blood flow to the peripheral regions. In this way, wide swings in the pressure and flow generated by the contraction and relaxation of the heart are prevented. A system having rigid tubes could not effect the necessary dampening in blood flow and pressure.

The compliance of the present invention may be produced and is adjustable by varying the materials from which each component of the present invention is made. For example, a graft may be made having a compliance which more closely resembles that of the vessel in which it is to be implanted by using, as a source for the biological compliant tissue, the same type of vessel. A graft made from processed arterial tissue will have a compliance and mechanical strength that, without little more, will more closely resemble that of the arterial tissue in which it is implanted. On the other hand, if the biological component is made from processed venous tissue, a graft can be made which is adjustable from a naturally very compliant state to a less compliant state according to the character of the synthetic component positioned over the resultant biological component. Similarly, the compliance of the outer component, and thereby the entire graft, may be varied by varying the durometer hardness of the synthetic material chosen to make the outer component.

The compliance of the present invention may be produced and is adjustable by varying also the method by which the outer synthetic component is made. For example, the greater the angle at which the synthetic fibers of the outer synthetic component are wound in relationship to the axis of the cylindrical-shaped synthetic component, the less compliant the component. Also, the thinner the wall of the synthetic structure is made, the more compliant the component will be. Additionally, the higher the Young's modulus of the material comprising the outer material, the less compliant the vessel.

A graft whose compliance matches that of the host vessel will be to maintain the normal pattern of blood flow and pressure through the vessel. More specifically, with regard to arterial applications, the graft may be adjusted to have a compliance which mimics the elastic recoil of natural arteries, thereby dampening the oscillatory nature of the blood flow and pressure as the blood exits the left ventricle.

The present invention also allows, advantageously, the dimensions of the graft to be varied to better match the site of implantation. A graft having a lumen equal to that of the host vessel may be produced by choosing natural tissue having a similarly-sized lumen from which the inner biological component may be made. A graft having an outer diameter equal to that of the vessel to which the graft will be joined may be produced by choosing natural tissue for processing that has an outer diameter less than that of the vessel to which the graft is to be joined and by adding a sufficient number of synthetic strand layers to make up the difference.

As the inner biological component is made from a generally porous biocompatible matrix and the synthetic component is made from a porous network of strands, the resultant porosity of the graft promotes recellularization by host connective tissue. Such tissue ingrowth within the porous graft is desirable in that it ensures a secure attachment between the graft and surrounding tissue.

Other advantages of the present invention include - in comparison to wholly biological and other biosynthetic grafts - the greater ability of the two component graft of the present invention to accept and retain sutures within the synthetic component. Also, as the present invention can be formed with the biological component in a dehydrated state, the graft may be stored for long periods of time and at room temperature without the use of preservative solutions commonly used with wholly biological or other biosynthetic grafts.

It is accordingly, a general object of the present invention to provide an improved graft.

Another object of the present invention is to provide an improved graft assembly made from a composite of biological materials and synthetic material that renders the graft hemocompatible and biocompatible.

It is also an object of this invention to provide an improved graft assembly having a inner graft component made of biological material whose natural compliance is not substantially impaired by the inner component's reinforcement with synthetic material.

It is an additional object of this invention to provide an improved graft whose compliance is generally adjustable.

Another object of this invention is to provide a composite biosynthetic graft and a method of making same.

These and other objects, features and advantages of this invention will be clearly understood through a consideration of the following detailed description.

## Brief Description of the Drawings

In the course of this description, reference will be made to the attached drawings, wherein:

Figure 1 is an elevational perspective view illustrating an embodiment of a composite vascular graft according to the present invention with an outer component positioned over an inner component and with the inner component extended to illustrate its structure;

Figure 2 is a cross sectional view of the composite vascular graft according to the present invention; and

Figure 3 is an elevational perspective view illustrating the outer component as positioned over the inner component.

## Description Of The Particular Embodiments

The present invention typically is a composite graft - generally designated as 20 - comprised of a inner biological component 30 made from natural vascular tissue and an outer synthetic component 40 made from synthetic materials according to known methods. The inner component 30 will be described first.

The methods by which the inner component 30 may be fabricated include those disclosed in U.S. Patents No. 4,776,853 to Klement et al. and 4,801,299 to Brendel et al. In one fabrication method disclosed in this patent, a starting material, such as natural body tissues is surgically removed from an animal (e.g., pig, cow, dog, horse, etc.) or man. Preferably, while immune reactions to the material can be lessened by using tissue from the same species, for purposes of implantation within man, the starting material is largely drawn from other mammals. As detailed in greater detail in U.S. Patent No. 4,776,853, the tissue is processed according to the following steps. The tissue as surgically isolated is subjected to a hypotonic buffer solution containing suitable protease inhibitors and active amounts of antibiotics. To remove cytoplasmic components and soluble extra- cellular matrix components, the vessels are subjected to a buffered high salt solution containing a non-ionic detergent, protease inhibitors, and antibiotics.

Following the washing of the resultant tissue with distilled water and its equilibriation with a buffered saline solution, the nuclear material of the tissue, and even entrapped bacteria and viruses, is digested with a mixture of purified, protease free, deoxyribonuclease and ribonuclease enzymes. The resultant tissue is subjected to anionic detergent then washed with distilled water, and finally stored in a physiologic saline antibiotic solution.

Because they contribute minimally to the mechanical properties of the vessel and because they may induce an immune reaction, the cell membranes, cytoplasm, nuclear material, and serum components are thereby removed from the tissue. A dense biological matrix of collagen and elastin fibers and glycosaminoglycans found in the tissue's basement membrane remains intact. On a microscopic level, the resultant matrix comprises residual basement membrane and provides a smooth membrane-like surface that is subtended by the internal lamina and underlying collagen fibers to form in profile a fibrillary component. Immunohistochemically, the luminal surface of the matrix contains basal lamina components, including type IV collagen.

The biological matrix in this resultant state may be mounted directly onto a metal mandrel so that the matrix may dry naturally or through freeze drying to form an inner biological component 30. The mandrel chosen to receive the matrix component should have an outer diameter that is equal to or slightly less than the diameter of the lumen 25 of the intended generally cylindrical biological component 30 formed thereby.

The methods by which the synthetic component 40 may be fabricated include those disclosed in U.S. Patent No. 4,475,972 to Wong and/or those disclosed in U.S. Patent No. 4,738,740 to Pinchuk and Martin. In one fabrication method disclosed in the Wong patent, termed "solution processing", a viscous solution - formed by dissolving a biocompatible polymeric material, such as biocompatible polyurethane, in a suitable solvent such as dimethyl formamide - is distributed under pressure out of one or more orifices to form one or more continuous fibers. In one embodiment of the present invention, the fibers are placed directly over the outer surface 32 of the biological component 30, in a dehydrated state and as held onto a rotating mandrel. With the reciprocation of the distributor or spinnerette from one axial end of the mandrel to the other, a non-woven layer of synthetic fiber is layered onto the biological component. In another embodiment, the synthetic fibers are laid directly onto a rotating mandrel to form a cylindrically-shaped synthetic component 40. As the outer synthetic component 40 is positioned over the dehydrated inner biological component 30 to form the composite graft 20 of the present invention, the mandrel onto which the synthetic fibers are layered must have an outer diameter generally equal to or slightly larger than the diameter of the outer surface 32 of the biological component 30. In one of the fabrication methods disclosed in Pinchuk and Martin, the extruded fibers are wound or spun on to a mandrel while being intermittently subjected to electrostatic charge conditions. The mandrel may be charged as may be the distributor or spinnerette. Such electrostatic conditions aid in allowing fibers broken during spinning to be reattached at or near its point of breakage and provide vascular grafts with good interfiber bonding and closely controlled pore size.

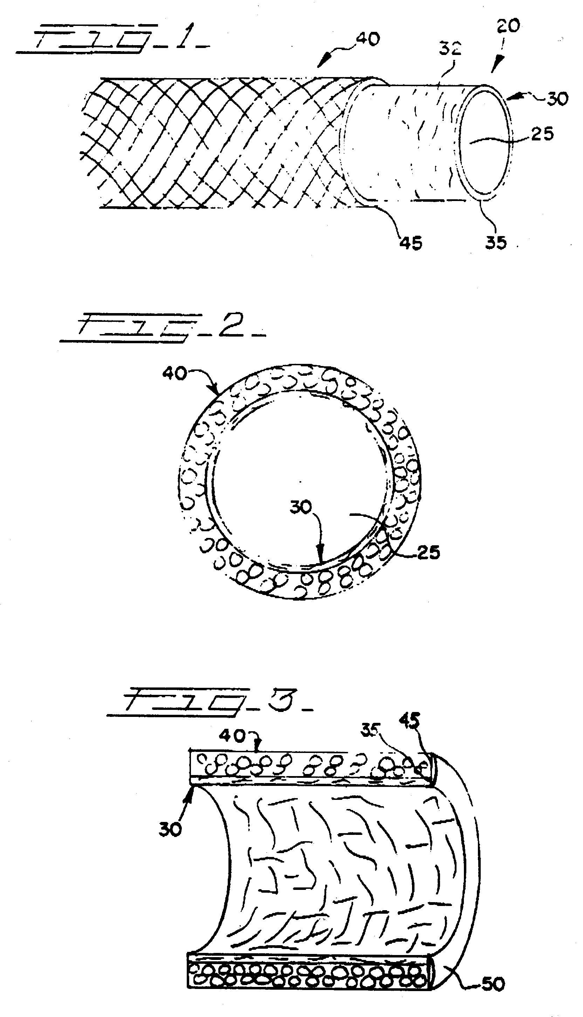

The biological component 30 and the synthetic component 40 may be united according to a number of methods. For example, after the hydration of the biological component 30, the two components may be held in place by mechanical elements. Such mechanical elements may include sutures, clips, etc. that join one or both ends of the two components together at one or both of their ends. The synthetic component 40 may be held in position over the biological component 30 also by "friction fit"; that is, the diameter of the outer surface 32 is less than but approximately equal to that of the inner surface 42 of the outer component 40, such that the outer component 40 as positioned over the inner component 30 is held in place by the friction between the two components. Alternatively, the two components may be joined together by adhesives such as proteinaceous adhesives or cyanoacrylate cement or fibrin glue. In Figure 3, an embodiment of the component graft assembly 20 is shown in which the outer synthetic component 40 is positioned over the inner biological component 30 and held by an adhesive layer 50 affixing the end portions 35, 45 respectively of each component 30, 40 to each other.

As the biological component 30 of the composite graft 20 is dehydrated, such as through freeze-drying or natural dehydration, the composite graft 20 may be stored for long periods of time at room temperature. This is a distinct advantage over other biological and biosynthetic grafts which must generally be stored in preservative solutions.

Advantageously, by the present invention, a composite graft 20 may be formed having dimensional characteristics equal to that of the vessel in which the graft is implanted. For example, the vessels of the arterial system closer to the heart characteristically have a larger lumen, a thicker layer of elastic and collagenous fibers, and a thicker surrounding layer of circular longitudinal smooth muscle fibers in comparison to those arterial vessels farther from the heart. A graft 20 according to the present invention may be formed for arterial implantation closer to the heart by choosing as a starting material a vessel having an appropriately larger lumen, processing it so that the resultant biological component 30 forms a similarly sized lumen 25, winding a sufficient number of synthetic strands to form an outer synthetic component 40 that will provide mechanical strength equal to that of the artery to be replaced or bypassed, and positioning the biological component 30 with the synthetic component 40 to form the necessary complaints, yet mechanically strong graft 20.

Further advantageously by the present invention, a composite graft 20 may be formed having compliance characteristics appropriate for any application. For example, the compliance of the composite graft 20 may be adjusted by varying the dimensions of and/or number of the fibers from which the outer synthetic component 40 is made. A graft 20 having an outer synthetic component 40 made from fibers of a smaller diameter and/or fewer in number will generally be more compliant than a graft having an outer component made from fibers of a larger diameter and/or greater in number.

The compliance of the composite graft 20 may be adjusted also by varying the number of non-interwoven fiber layers by which the outer synthetic component 40 is formed. Composite graft compliance may be adjusted also by varying the synthetic materials from which the outer component 40 is formed: synthetic materials having greater durometer hardness will produce a less compliant outer component.

The compliance of the outer synthetic component 40 is adjustable also by the angle at which the synthetic fibers are laid down on the rotating mandrel - the greater the angle at which the fibers are laid down in relationship to the angle of the mandrel axis, the less compliant the component.

In all cases, the actual compliance of the graft 20 that is required will be dictated by the vascular location in which the graft 20 is to be implanted and the resultant range of pressure which the graft 20 is expected to maintain.

When the outer synthetic component 40 is formed separate from and not directly onto the outer surface 32 of the inner biological component 30, the outer synthetic component 40, after positioning over the inner biological component 30, may be fixed to it with the use of mechanical means or adhesive means, the choice of which will alter also the compliance characteristics of the composite graft 20.

The other performance characteristics of the graft 20 may be optimized according to the application requirements. For example, the kink resistance - that is, the ability of the graft 20 to be bent without the resultant great reduction in the inner cross-sectional area of the graft 20 - may be increased by the winding of the synthetic fibers at a greater angle. Such a graft 20 may be used in locations which are generally subjected to more flexing. The shape of the pores and the overall porosity of the graft 20 may also be adjusted to meet the application need. For example, the smaller the angle of at which the synthetic fibers are wound, the smaller the pore size, and the lower the resultant porosity. Accordingly, by the present invention, a composite graft 20 may be formed which has a spun, non-woven outer synthetic network having a high degree of porosity and by which the natural in-growth of tissue into the graft 20 and thereby the greater stable incorporation of the graft 20 in the implant site will be promoted.

The composite biosynthetic vascular graft 20 as formed may be held implanted in the desired vascular location through conventional methods, such as suturing. The use of synthetic materials to form the outer synthetic component 40 facilitates a greater retention of the sutures by the graft in comparison to biological and other biosynthetic grafts.

It will be understood that the embodiments of the present invention as described are illustrative of some of the applications of the principles of the present invention. Modifications may be made by those skilled in the art without departure from the spirit and scope of the invention.

**Claims**

1. A composite vascular graft for implantation within a host, comprising:

   a biological component made from generally non-absorbable extracellular matrix, said extracellular matrix including collagenous and elastic fibers processed from natural tissue, said extracellular matrix shaped to form a generally elongated cylindrical shape having a lumen through which blood may flow;

   a synthetic component made from non-interwoven windings of biocompatible synthetic material by which porosity and compliance may be adjustable and said synthetic component is positioned generally over an outer surface of said biological component; and

   said biological component and said synthetic component cooperate to form a compliant porous composite vascular graft having compliance and porosity characteristics which are adjustable and having biocompatibility characteristics which facilitate the ingrowth of cells and tissue from, and thereby the incorporation of the graft within the host.

2. The composite vascular graft according to claim 1, wherein said extracellular matrix is extracted from natural venous tissue.

3. The composite vascular graft according to claim 1, wherein said extracellular matrix is formed by extracting cells from natural arterial tissue.

4. The composite vascular graft according to claim 1, wherein said extracellular matrix includes a basement membrane of natural vascular tissue.

5. The composite vascular graft according to claim 1, wherein said windings are positioned over said biological component by directly layering said windings onto said outer surface of said biological network.

6. The composite vascular graft according to claim 1, wherein said synthetic network is held in position over said biological network by mechanical means

7. The composite vascular graft according to claim 6, wherein said mechanical means include sutures through ends of said synthetic network and said biological network.

8. The composite vascular graft according to claim 1, wherein said synthetic network is held in position over said biological network by adhesive means.

9. The composite vascular graft according to claim 1, wherein said windings are positioned over a biological network having been dehydrated.

10. The composite vascular graft according to claim 9, wherein said biological network is freeze-dried.

11. The composite vascular graft according to claim 1, wherein said synthetic network is held in position over said biological network by friction fit.

12. The composite vascular graft according to claim 1, wherein said synthetic component is made by the winding of said biocompatible synthetic materials under the influence of electrostatic charges.

13. A composite vascular graft prepared by a process comprising the steps of:
(a) extracting absorbable cellular materials from naturally-occurring vascular tissue to produce a biological matrix;
(b) shaping said biological matrix over a mandrel to form a generally elongated cylindrical-shaped compliant biological component having a lumen therethrough, said lumen having an inner diameter generally equal to the outer diameter of said mandrel;
(c) dehydrating said biological component;
(d) winding non-interwoven strands of biocompatible synthetic material over a mandrel to form a synthetic component; and
(e) positioning said synthetic component over said biological component to form a component vascular graft having compliance characteristics and size characteristics that are adjustable.

14. The composite vascular graft prepared by the process according to claim 13, wherein said extracting step provides a biological component in which the basement membrane of said naturally-occurring vascular tissue largely remains intact after said extracting step.

15. The composite vascular graft prepared by the process according to claim 13, wherein the dehydrating step is performed by freeze-drying.

16. The component vascular graft prepared by the process according to claim 13, wherein the winding step and the positioning step are carried out simultaneously.

17. The composite vascular graft prepared by the process according to claim 13, wherein said strands of biocompatible synthetic material are wound under the influence of electrostatic charges.

18. A composite vascular graft prepared by a process comprising the steps of:
(a) shaping biological matrix including basement membrane, collagen, and elastin from naturally-occurring tissue over an mandrel to form an elongated cylindrical biological component having an outer surface and a lumen therethrough;
(b) dehydrating said biological component;
(c) winding non-interwoven strands of biocompatible synthetic material over a mandrel to form a synthetic component having non-interwoven layers by which compliance and size of said synthetic may be adjusted, said mandrel having an outer diameter equal to or slightly larger than said outer surface of said biological component; and
(d) positioning said synthetic component over said biological component to form a composite vascular graft whose compliance characteristics and size characteristics are generally adjustable and through the lumen of said graft as implanted in vascular tissue blood may flow.

19. The process according to claim 18, wherein said dehydrating step is performed by freeze drying.

20. The process according to claim 18, further including a step of joining the biological component to the synthetic component by mechanical elements.

21. The process according to claim 18, further including a step of joining the biological component to the synthetic component by adhesives.

22. A method for forming a composite vascular graft, which method comprises:

removing absorbable elements from naturally-occurring vascular tissue to form an extracellular matrix including collagenous and elastinous fibers;

dehydrating said extracellular matrix over a mandrel to form a porous lower component have a lumen therethrough; and

winding strands of compliant biocompatible synthetic material in layers over said lower component to form a composite vascular graft having an upper layer, said winding being carried out without interweaving and by laying said stands in a direction different from and generally opposite to the direction of an immediately previous layer and by which compliance and size of said graft may be adjusted.

23. The method according to claim 22, wherein said dehydrating step is performed by freeze drying.

FIG_1_

FIG_2_

FIG_3_